(19) **Europäisches Patentamt / European Patent Office / Office européen des brevets**

(11) **EP 2 826 494 B1**

(12) **EUROPEAN PATENT SPECIFICATION**

(45) Date of publication and mention of the grant of the patent:
**06.01.2021 Bulletin 2021/01**

(21) Application number: **13761579.5**

(22) Date of filing: **12.03.2013**

(51) Int Cl.:
*A61L 27/00* *(2006.01)*

(86) International application number:
**PCT/JP2013/056849**

(87) International publication number:
**WO 2013/137268 (19.09.2013 Gazette 2013/38)**

(54) **METHOD FOR PRODUCING TISSUE REPAIR MATERIAL**

VERFAHREN ZUR HERSTELLUNG VON GEWEBEREPARATURMATERIAL

PROCÉDÉ DE FABRICATION D'UN MATÉRIAU DE RÉPARATION DES TISSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2012 JP 2012055020**

(43) Date of publication of application:
**21.01.2015 Bulletin 2015/04**

(73) Proprietor: **Fujifilm Corporation**
**Minato-ku**
**Tokyo 106-8620 (JP)**

(72) Inventors:
• **OYA, Shoji**
 **Ashigarakami-gun**
 **Kanagawa 258-8577 (JP)**
• **SAKAI, Hidekazu**
 **Ashigarakami-gun**
 **Kanagawa 258-8577 (JP)**
• **KAWAKAMI, Ai**
 **Ashigarakami-gun**
 **Kanagawa 258-8577 (JP)**
• **HIRATSUKA, Takahiro**
 **Ashigarakami-gun**
 **Kanagawa 258-8577 (JP)**

• **FUSHIMI, Hideo**
 **Ashigarakami-gun**
 **Kanagawa 258-8577 (JP)**
• **YAMAGUCHI, Kazuhiro**
 **Ashigarakami-gun**
 **Kanagawa 258-8577 (JP)**
• **NISHIMURA, Ichiro**
 **Santa Monica, California 90405 (US)**
• **OONO, Yoshitaka**
 **Ashigarakami-gun**
 **Kanagawa 258-8577 (JP)**
• **OGURA, Izumi**
 **Ashigarakami-gun**
 **Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 478 923      EP-A1- 2 543 398**
**WO-A1-2011/027850      WO-A1-2011/108537**

Remarks:
The file contains technical information submitted after the application was filed and not included in this specification

SEQUENCE LISTING

<110> FUJIFILM CORPORATION

<120> Method of Producing Tissue Repairing Material

<130> FS-F05064-00

<150> JP2012-055020
<151> 2012-03-12

<160> 1

<170> PatentIn version 3.4

<210> 1
<211> 571
<212> PRT
<213> Artificial

<220>
<223> CBE3

<400> 1

```
Gly Ala Pro Gly Ala Pro Gly Leu Gln Gly Ala Pro Gly Leu Gln Gly
1               5               10              15

Met Pro Gly Glu Arg Gly Ala Ala Gly Leu Pro Gly Pro Lys Gly Glu
            20              25              30

Arg Gly Asp Ala Gly Pro Lys Gly Ala Asp Gly Ala Pro Gly Ala Pro
        35              40              45

Gly Leu Gln Gly Met Pro Gly Glu Arg Gly Ala Ala Gly Leu Pro Gly
    50              55              60

Pro Lys Gly Glu Arg Gly Asp Ala Gly Pro Lys Gly Ala Asp Gly Ala
65              70              75              80

Pro Gly Lys Asp Gly Val Arg Gly Leu Ala Gly Pro Ile Gly Pro Pro
            85              90              95

Gly Glu Arg Gly Ala Ala Gly Leu Pro Gly Pro Lys Gly Glu Arg Gly
        100             105             110

Asp Ala Gly Pro Lys Gly Ala Asp Gly Ala Pro Gly Lys Asp Gly Val
    115             120             125

Arg Gly Leu Ala Gly Pro Ile Gly Pro Pro Gly Pro Ala Gly Ala Pro
    130             135             140

Gly Ala Pro Gly Leu Gln Gly Met Pro Gly Glu Arg Gly Ala Ala Gly
145             150             155             160
```

Leu Pro Gly Pro Lys Gly Glu Arg Gly Asp Ala Gly Pro Lys Gly Ala
165                    170                    175

Asp Gly Ala Pro Gly Lys Asp Gly Val Arg Gly Leu Ala Gly Pro Pro
180                    185                    190

Gly Ala Pro Gly Leu Gln Gly Ala Pro Gly Leu Gln Gly Met Pro Gly
195                    200                    205

Glu Arg Gly Ala Ala Gly Leu Pro Gly Pro Lys Gly Glu Arg Gly Asp
210                    215                    220

Ala Gly Pro Lys Gly Ala Asp Gly Ala Pro Gly Ala Pro Gly Leu Gln
225                    230                    235                    240

Gly Met Pro Gly Glu Arg Gly Ala Ala Gly Leu Pro Gly Pro Lys Gly
245                    250                    255

Glu Arg Gly Asp Ala Gly Pro Lys Gly Ala Asp Gly Ala Pro Gly Lys
260                    265                    270

Asp Gly Val Arg Gly Leu Ala Gly Pro Ile Gly Pro Pro Gly Glu Arg
275                    280                    285

Gly Ala Ala Gly Leu Pro Gly Pro Lys Gly Glu Arg Gly Asp Ala Gly
290                    295                    300

Pro Lys Gly Ala Asp Gly Ala Pro Gly Lys Asp Gly Val Arg Gly Leu
305                    310                    315                    320

Ala Gly Pro Ile Gly Pro Pro Gly Pro Ala Gly Ala Pro Gly Ala Pro
325                    330                    335

Gly Leu Gln Gly Met Pro Gly Glu Arg Gly Ala Ala Gly Leu Pro Gly
340                    345                    350

Pro Lys Gly Glu Arg Gly Asp Ala Gly Pro Lys Gly Ala Asp Gly Ala
355                    360                    365

Pro Gly Lys Asp Gly Val Arg Gly Leu Ala Gly Pro Pro Gly Ala Pro
370                    375                    380

Gly Leu Gln Gly Ala Pro Gly Leu Gln Gly Met Pro Gly Glu Arg Gly
385                    390                    395                    400

Ala Ala Gly Leu Pro Gly Pro Lys Gly Glu Arg Gly Asp Ala Gly Pro

```
                    405                    410                    415


        Lys Gly Ala Asp Gly Ala Pro Gly Ala Pro Gly Leu Gln Gly Met Pro
                    420                    425                    430


        Gly Glu Arg Gly Ala Ala Gly Leu Pro Gly Pro Lys Gly Glu Arg Gly
                    435                    440                    445


        Asp Ala Gly Pro Lys Gly Ala Asp Gly Ala Pro Gly Lys Asp Gly Val
                450                    455                    460


        Arg Gly Leu Ala Gly Pro Ile Gly Pro Pro Gly Glu Arg Gly Ala Ala
        465                    470                    475                    480


        Gly Leu Pro Gly Pro Lys Gly Glu Arg Gly Asp Ala Gly Pro Lys Gly
                            485                    490                    495


        Ala Asp Gly Ala Pro Gly Lys Asp Gly Val Arg Gly Leu Ala Gly Pro
                    500                    505                    510


        Ile Gly Pro Pro Gly Pro Ala Gly Ala Pro Gly Ala Pro Gly Leu Gln
                    515                    520                    525


        Gly Met Pro Gly Glu Arg Gly Ala Ala Gly Leu Pro Gly Pro Lys Gly
                530                    535                    540


        Glu Arg Gly Asp Ala Gly Pro Lys Gly Ala Asp Gly Ala Pro Gly Lys
        545                    550                    555                    560


        Asp Gly Val Arg Gly Leu Ala Gly Pro Pro Gly
                            565                    570
```

**Description**

Technical Field

The present invention relates to a method of producing a tissue repair material.

Background Art

At present, practical application of regenerative medicine, in which attempts are made to regenerate body tissues and organs that are functionally impaired or dysfunctional, is in progress. Regenerative medicine is a new medical technology in which, for body tissues that cannot be recovered by the natural healing ability of the living body alone, morphologies and functions that are similar to those of the original tissues are regenerated using 3 factors, that is, cells, scaffolds, and growth factors.

In the field of regeneration medicine, collagen and gelatin, which are highly biocompatible, are sometimes used for the purpose of supporting recovery or regeneration of tissues by cells. In particular, collagen and gelatin are sometimes used for regeneration of tissues with three-dimensional structures such as bones and skins. Therefore, various improvements are being made in order to achieve favorable regeneration of tissues.

For example, WO2011/027850 discloses a bone regeneration agent and a bone filling preparation which contain a recombinant gelatin and is capable of promoting bone regeneration by the filling carrier itself.

Japanese Patent Application Laid-Open (JP-A) No. H8-196618 discloses a cell-invasive collagen preparation containing a collagen matrix in which many pores are present.

SUMMARY OF INVENTION

Problem to be Solved by the Invention

However, all the techniques described above still need to be improved from the viewpoint of obtaining a tissue repair material that promotes bone regeneration.

An object of the invention is to provide a method of producing a tissue repair material, by which a tissue repair material having favorable tissue regeneration ability can be obtained.

Means for Solving the Problems

The invention is defined by the claims.

Effect of the Invention

By the invention, a method of producing a tissue repair material, by which a tissue repair material having favorable tissue regeneration ability can be obtained, can be provided.

DESCRIPTION OF EMBODIMENTS

[Method of Producing Tissue Repair Material]

In the invention, "tissue repair material" means a material that contributes, by being embedded in a living body, to formation of a tissue in the site where the material is embedded. The tissue repair material may contain cells or may not contain cells. Further, the tissue repair material may contain a component that promotes reaction of a living body, such as a growth factor or agent or may not contain the component. Further, the tissue repair material may be mixed, or prepared into a composite, with an inorganic material such as hydroxyapatite.

Examples of the "tissue repair material" in the invention include not only materials that contribute to formation of a normal tissue that is normally present in the embedded site, but also materials that promote formation of an abnormal tissue such as a scar tissue.

The method of producing a tissue repair material of the invention comprises: (a) obtaining a gelatin-containing intermediate that contains a gelatin and has a mesh structure, using a gelatin solution in which the gelatin is dissolved in an aqueous medium (hereinafter referred to as the intermediate-production step); (b) drying the gelatin-containing intermediate (hereinafter referred to as the drying step); and (c) cross-linking the gelatin before or after the drying of the gelatin-containing intermediate (hereinafter referred to as the cross-linking step).

In the present production method, a gelatin-containing intermediate that contains a gelatin and has a mesh structure is first obtained, and the obtained intermediate is then dried to obtain a tissue repair material. Therefore, a tissue repair material retaining a shape appropriate for tissue repair can be obtained.

In the present description, the term "step" means not only an independent step, but also a step that cannot be distinguished from other steps as long as a predetermined action of the step is achieved.

In the description, the range of values represented using "to" means the range in which the values described before and after "to" are included as the lower limit value and the upper limit value, respectively.

In the invention, in cases where a plurality of substances corresponding to a single component are present in a composition, the amount of component in the composition means the total amount of the plurality of substances present in the composition, unless otherwise specified.

In the invention, the amino acid sequence constituting a polypeptide may be represented using single-letter codes (for example, "G" represents a glycine residue) or three-letter codes (for example, "Gly" represents a glycine residue), which are well known in the art.

In the invention, the tissue repair material means a tissue repair material obtained after the drying step and the cross-linking step, and the gelatin-containing intermediate means a material that has not been processed through at least one of the drying step or the cross-linking step.

The invention is described below.

(a) Intermediate-production Step

In the intermediate-production step, a gelatin-containing intermediate that contains a gelatin and has a mesh structure is obtained using a gelatin solution in which the gelatin is dissolved in an aqueous medium.

The gelatin means a polypeptide comprising 6 or more contiguous units of a sequence represented by Gly-X-Y, and may comprise one or more amino acid residues in addition to the sequence(s) represented by Gly-X-Y Each of the sequence(s) represented by Gly-X-Y is a sequence corresponding to the amino acid sequence derived from a partial amino acid sequence of collagen, and repeating of this sequence means a sequence characteristic to collagen.

The plurality of Gly-X-Y may be the same as or different from each other. The X and Y in the Gly-X-Y sequence are independently represented in each repeating unit, and may be the same as or different from each other. In Gly-X-Y, Gly represents a glycine residue, and each of X and Y means an arbitrary amino acid residue other than a glycine residue. As each of X and Y, imino acid residues, that is, proline residues and/or oxyproline residues, are preferably contained in a large amount. The content of theimino acid residues preferably accounts for from 10% to 45% of the whole gelatin. The content of Gly-X-Y in the gelatin is preferably 80% or higher, more preferably 95% or higher, most preferably 99% or higher with respect to the whole gelatin.

The gelatin may be a natural-type gelatin, or a mutant gelatin having at least one amino acid residue different from that of a natural-type gelatin. The gelatin is preferably a recombinant gelatin obtained by introduction, into an appropriate host, of a base sequence or amino acid sequence prepared by altering one or more bases or amino acid residues in a gene encoding the collagen comprising 6 or more contiguous units of a sequence represented by Gly-X-Y, and expression of the sequence in the host, by ordinary methods. By using such a recombinant gelatin, the tissue repair ability can be increased, and, compared to cases where a natural-type gelatin is used, various properties can be expressed. For example, disadvantageous effects such as rejection reaction by the living body can be avoided, which is advantageous.

Examples of the recombinant gelatin that may be particularly preferably used include recombinant gelatins disclosed in EP 1014176 A2, US 6992172, WO2004/85473, WO2008/103041, JP-A2010-519293, JP-A 2010-519252, JP-A 2010-518833, JP-A 2010-519251, WO2010/128672, and WO2010/147109.

The recombinant gelatin preferably has a molecular weight of from 2 kDa to 100 kDa, more preferably from 5 kDa to 90 kDa, still more preferably from 10 kDa to 90 kDa.

In view of biocompatibility, the recombinant gelatin preferably further contains a cell adhesion signal, and 2 or more cell adhesion signals are more preferably present in each molecule of the recombinant gelatin. Examples of the cell adhesion signal include RGD sequences, LDV sequences, REDV sequences, YIGSR sequences, PDSGR sequences, RYVVLPR sequences, LGTIPG sequences, RNIAEIIKDI sequences, IKVAV sequences, LRE sequences, DGEA sequences, and HAV sequences. Preferred examples of the cell adhesion signal include the RGD sequences, YIGSR sequences, PDSGR sequences, LGTIPG sequences, IKVAV sequences and HAV sequences, and particularly preferably include RGD sequences. Among the RGD sequences, ERGD sequence is still more preferable.

In the recombinant gelatin, RGD sequences are preferably arranged such that the number of amino acid residues present between RGDs is from 0 to 100, more preferably from 25 to 60. The RGD sequences are preferably unevenly arranged such that the number of amino acid residues placed therebetween is within this range.

The ratio of RGD sequences with respect to the total number of amino acid residues in the recombinant gelatin is preferably at least 0.4%, and, in cases where the recombinant gelatin contains 350 or more amino acid residues, each stretch of 350 amino acid residues preferably contains at least 1 RGD sequence.

The recombinant gelatin contains preferably at least 2 RGD sequences, more preferably at least 3 RGD sequences, still more preferably at least 4 RGD sequences, per 250 amino acid residues. The sequence of the recombinant gelatin is preferably the followingembodiment: (1) the sequence contains neither a serine residue nor a threonine residue; (2) the sequence contains none of a serine residue, threonine residue, asparagine residue, tyrosine residue and cysteine residue; or (3) the sequence does not contain the amino acid sequence represented by Asp-Arg-Gly-Asp. The recombinant gelatin may be one that satisfies any one of these preferred embodiments of the sequences (1) to (3), or any combination of two or more of these embodiments.

The recombinant gelatin may be partially hydrolyzed.

The recombinant gelatin preferably has a repeating structure of A-[(Gly-X-Y)$_n$]$_m$-B. m represents from 2 to 10, preferably represents 3 to 5. Each of A and B represents an arbitrary amino acid or amino acid sequence. n represents from 3 to 100, preferably from 15 to 70, more preferably from 50 to 60.

The recombinant gelatin is preferably represented by the formula: Gly-Ala-Pro-[(Gly-X-Y)$_{63}$]$_3$-Gly (wherein each of the 63 Xs independently represents any amino acid residue; each of the 63 Ys independently represents any amino acid residue; and the 3 units of (Gly-X-Y)$_{63}$ may be the same as or different from each other).

To the repeating units in the recombinant gelatin, a plurality of sequence units of naturally occurring collagen are preferably bound. Preferred examples of the naturally occurring collagen herein include type I, type II, type III, type IV and type V. The collagen may be more preferably type I, type II or type III. Preferred examples of the origin of collagen include human, horse, pig, mouse and rat. The origin is more preferably human. The isoelectric point of the recombinant gelatin is preferably from 5 to 10, more preferably from 6 to 10, still more preferably from 7 to 9.5.

Preferred examples of the embodiment of the recombinant gelatin include: (1) the gelatin is not deaminated; (2) the gelatin does not contain procollagen; (3) the gelatin does not contain telopeptide; and (4) the gelatin is a substantially pure material for collagen, prepared by using nucleic acid encoding a naturally occurring collagen. The recombinant gelatin may be one that satisfies any one of these preferred embodiments (1) to (4), or any combination of two or more of these embodiments.

In view of achieving high tissue repair ability, the recombinant gelatin may be preferably any of (A) to (C) below:

(A) the polypeptide of SEQ ID NO.1:

GAP(GAPGLQGAPGLQGMPGERGAAGLPGPKGERGDAGPKGADGAPGAPGLQGMP

GERGAAGLPGPKGERGDAGPKGADGAPGKDGVRGLAGPIGPPGERGAAGLPGPKGE

RGDAGPKGADGAPGKDGVRGLAGPIGPPGPAGAPGAPGLQGMPGERGAAGLPGPKG

ERGDAGPKGADGAPGKDGVRGLAGPP)$_3$G (SEQ ID NO:1);

(B) a polypeptide having a partial sequence with a sequence identity of 80% or higher to the partial amino acid sequence consisting of from the 4th to 192nd amino acid residues in the amino acid sequence of (A), and having tissue repair ability; or

(C) a polypeptide having the same amino acid sequence as the amino acid sequence of (A) except that one or several amino acids are deleted, substituted or added, and having tissue repair ability.

The sequence identity in (B) may be more preferably 90% or higher, still more preferably 95% or higher, in view of tissue repair ability of the recombinant gelatin.

The "sequence identity" in the present invention means a value calculated by the following formula.

$$\% \text{ Sequence identity} = [(\text{number of identical residues}) / (\text{alignment length})] \times 100$$

The partial amino acid sequence in the sequence of (B) is a partial amino acid sequence corresponding to the repeating unit in the sequence of SEQ ID NO. 1. In cases where a plurality of partial amino acid sequences each corresponding to the repeating unit are present in the polypeptide of (B), the polypeptide may be a polypeptide having one, preferably two or more, repeating unit(s) each having a sequence identity of 80% or higher.

The polypeptide defined by (B) preferably comprises a partial amino acid sequence having a sequence identity of 80% or higher to the partial amino acid sequence corresponding to the repeating unit in an amount of 80% or higher in terms of the total number of amino acid residues of the partial amino acid sequence, with respect to the total number of amino acid residues in the polypeptide.

The length of the polypeptide defined by (B) may be from 151 to 2260 amino acid residues. In view of degradability after the cross-linking, the number of amino acid residues is preferably 193 or more, and, in view of stability, the number of amino acid residues is preferably 944 or less. The number of amino acid residues is more preferably from 380 to 756.

The polypeptide defined in (C) may be a polypeptide having the same amino acid sequence as the amino acid sequence of (A) except that one or several amino acids are deleted, substituted or added, and having tissue repair ability.

The number of amino acid residues deleted, substituted or added in the polypeptide defined by (C) may be one or several. The number may be, for example, from 2 to 15, preferably from 2 to 5, although it varies depending on the total number of amino acid residues in the recombinant gelatin.

The recombinant gelatin may be produced by genetic recombination techniques known to those skilled in the art, and examples of methods for producing the recombinant gelatin include those described in EP 1014176 A2, US 6992172, WO2004/85473, and WO2008/103041. Specifically, a gene encoding the amino acid sequence of a predetermined recombinant gelatin is obtained and then incorporated into an expression vector to prepare a recombinant expression vector, followed by introducing the resulting vector to an appropriate host, to prepare a transformant. By culturing the thus obtained transformant in an appropriate medium, a recombinant gelatin is produced. By recovering the produced recombinant gelatin from the culture, a recombinant gelatin to be used in the invention can be prepared.

Evaluation of the tissue repair ability may be carried out using a tissue repair material obtained by using the recombinant gelatin described above. The method of evaluation varies depending on the tissue of interest.

For example, in cases of a bone tissue, the evaluation can be carried out based on the bone regeneration ability. The bone regeneration ability can be evaluated by preparing a bone defect having a predetermined size in the parietal bone of a rat, filling the bone defect with a predetermined amount of the tissue repair material, and then suturing the skin, followed by measuring the bone mass using a micro-CT on Week 4 after the operation, to calculate the ratio of the volume of the bone regenerated to the volume of the bone defect.

The tissue repair ability may also be evaluated based on bone bonding. The bone-bonding ability can be evaluated by filling a bone defect with a tissue repair material and then suturing the skin similarly to the evaluation of tissue repair ability, followed by HE staining of the head removed on Week 4 after the operation and investigation of the size of the area where the tissue repair material and new bone are in contact with each other without interposition of a fibrous tissue.

In cases where bone repair ability is found by either method of evaluation, the recombinant gelatin can be evaluated as having tissue repair ability.

The aqueous medium is not limited as long as it can dissolve gelatin and is applicable to a body tissue, and examples of the aqueous medium include aqueous media usually applicable in the art, such as water, physiological saline and phosphate buffer.

The content of gelatin in the gelatin solution is not limited as long as the gelatin can be dissolved at this content. For example, the content of gelatin in the gelatin solution is preferably from 0.5% by mass to 20% by mass, more preferably from 2% by mass to 16% by mass, still more preferably from 4% by mass to 12% by mass. In cases where the content is 0.5% by mass or higher, the strength tends to be high, and in cases where the content is 20% by mass or lower, a uniform mesh structure tends to be formed.

The gelatin solution in the intermediate-production step may be one already prepared, or a step of preparing the gelatin solution may be carried out before obtaining the gelatin-containing intermediate.

In the step of preparing a gelatin solution, the gelatin is provided as a material, and dissolved in the aqueous medium to prepare a gelatin solution. The gelatin as a material may be in the form of either a powder or solid.

The temperature during preparation of the gelatin solution is not limited, and may be a temperature usually used, such as a temperature of from 0°C to 60°C, preferably a temperature of from about 3°C to 30°C.

The gelatin solution may contain, if necessary, a component required in the later-described steps, such as a cross-linking agent or a component useful for giving a predetermined property to the tissue repair material.

The method of obtaining a gelatin-containing intermediate may be any method as long as the later-described predetermined voids can be formed.

Specifically, the gelatin-containing intermediate is preferably obtained by a method comprising stirring the gelatin solution to generate air bubbles in the gelatin solution (hereinafter referred to as the air bubble generation step) and then allowing the gelatin solution to gel (hereinafter referred to as the gelation step), or a method comprising cooling the recombinant gelatin solution to a temperature lower than the ice crystal formation temperature (hereinafter referred to as the ice crystal formation step).

In cases where the gelatin-containing intermediate is formed using a method comprising the air bubble generation step and the gelation step, the gelatin-containing intermediate tends to have voids having a spherical shape, while in cases where the gelatin-containing intermediate is formed using a method comprising the ice crystal formation step, the gelatin-containing intermediate tends to have voids having a columnar shape.

The conditions for stirring the gelatin solution applied to the air bubble generation step may be conditions under which air bubbles can be generated. In the invention, the stirring conditions under which air bubbles can be generated are defined as follows: the stirring Froude number Fr according to Formula (1) below is 2.0 or greater, and the stirring Reynolds number Re according to Formula (2) below is 6000 or less.

$$Fr = n^2d/g \ldots (1)$$

$$Re = \rho nd^2/\mu \ldots (2)$$

In both formulae, n represents the rate of stirring (per second), and d represents the diameter of the stirring blade (m). In Formula (1), g represents the gravitational acceleration ($m/s^2$). In Formula (2), $\rho$ represents the solution density ($kg/m^3$), and $\mu$ represents the solution viscosity (Pa·s).

The device used for stirring is not limited as long as stirring can be carried out at a stirring Froude number Fr of 2.0 or greater and a stirring Reynolds number Re of 6000 or less. Examples of the device include homogenizers, dissolvers, and homomixers.

Examples of the stirring conditions include stirring at a stirring rate of 1,400 rpm using a T. K. Homodisper Type 2.5 (manufactured by PRIMIX Corporation).

The temperature during stirring is preferably from 2°C to 50°C, more preferably from 5°C to 30°C in view of fluidity and maintaining air bubbles.

In the gelation step, gelation is carried out in a state where air bubbles are maintained in the gelatin solution in which air bubbles were generated. The gelation may be carried out by solidification by cooling at a temperature of, for example, from 1°C to 20°C.

In the method comprising the ice crystal formation step, the gelatin solution is cooled to a temperature lower than the ice crystal formation temperature. By this, the gelatin-containing intermediate containing ice crystals having appropriate sizes therein can be obtained. Since irregularity in the density of peptide chains of the recombinant gelatin is caused due to formed ice crystals and the gelatin-containing intermediate is solidified, mesh voids are formed in the gelatin-containing intermediate after disappearance of the ice crystals. The disappearance of ice crystals can be easily achieved by the drying step, which is described later. The mesh pore size in the gelatin-containing intermediate can be controlled by the ice crystal temperature or cooling time, or the combination thereof.

The ice crystal formation temperature means a temperature at which at least part of the gelatin solution is frozen. The ice crystal formation temperature varies depending on the concentration of solids including the recombinant gelatin in the gelatin solution, and may be generally -10°C or lower.

Preferably, the gelatin solution may be cooled to a temperature of from -100°C to -10°C, more preferably a temperature of from -80°C to -20°C. In cases where the temperature -100°C or higher, the mesh size tends to be sufficiently large, and in cases where the temperature is not more than -10°C, the mesh pore size of the gelatin-containing intermediate is highly uniform, and therefore favorable tissue repair ability tends to be exerted.

The period during which a temperature lower than the ice crystal formation temperature is maintained is preferably from 1 to 8 hours, more preferably from 1 to 6 hours, in view of uniformity.

Before the ice crystal formation step, the air bubble generation step may be carried out. Since, by this, ice crystals are formed after generation of air bubbles, a gelatin-containing intermediate having a better shape can be produced, and the tissue repair material obtained tends to have even better tissue repair ability. The conditions described above may be applied as they are to the air bubble generation step that is carried out before the ice crystal formation step.

By the intermediate-production step, a gelatin-containing intermediate that contains the gelatin and has a mesh structure can be obtained.

In the present description, the term "mesh structure" means a structure containing a large amount of voids therein, and is not limited to a planar structure. Examples of the mesh structure include not only those having a fibrous structure, but also those having a wall structure. The term means a structure of a construct constituted by a material containing gelatin, and does not mean a molecular-level structure such as a collagen fiber.

The term "having a mesh structure" means that a wall-shaped structure containing as a component the recombinant gelatin constituting the gelatin-containing intermediate forms voids having a pore size on the micrometer or larger scale, that is, having a pore size of 1 $\mu$m or larger.

The mesh shape in the gelatin-containing intermediate is not limited, and may be a two-dimensional structure such as a honeycomb, or a three-dimensional structure such as a cancellous bone. The cross-sectional shape of each mesh may be a polygon, circle, ellipse, or the like. The three-dimensional shape of the mesh in the gelatin-containing intermediate may be a column or a sphere.

In the gelatin-containing intermediate, communicating pores, in which voids are formed to communicate with each other, may be presented. By the presence of communicating pores, voids are connected from the outside to the deep inside of the gelatin-containing intermediate. Such connection of voids allows cells to disperse or spread into the inside of the porous body when the cells contact with the outside of the tissue repair material obtained from the gelatin-containing intermediate. Each communicating pore preferably has a size of not less than 10 μm for exertion of such a function.

The mesh pore size in the gelatin-containing intermediate is evaluated as the average of diameters in the longitudinal axis (longitudinal diameter). The average longitudinal diameter is evaluated as follows.

First, a dry intermediate obtained by drying the gelatin-containing intermediate is cut in the horizontal and vertical directions. Subsequently, each section is brought into close contact with an ink pad to perform staining, and an area of 2.0 mm × 2.0 mm is observed under a light microscope. In the observed area, among circumscribed rectangles to an area surrounded by the stained material, the circumscribed rectangle having the largest distance between two opposite sides of the rectangle is selected. The length of long side of the circumscribed rectangle having the largest distance between two opposite sides of the rectangle is measured for 50 mesh pores in the observation area in each of the horizontal section and the vertical section, and the average is defined as the average longitudinal diameter of meshe pores in the gelatin-containing intermediate.

Between the average of the longitudinal diameters of individual meshes in the horizontal section and the average of the longitudinal diameters of individual meshes in the vertical section as determined in the same manner as described above, the smaller value is defined as d1, and the other is defined as d2. The ratio d2/dl is referred to as the mesh aspect ratio. In cases where the mesh aspect ratio is from 1 to 3, the shape is regarded as "spherical", and in cases where the mesh aspect ratio is not within this range, the shape is regarded as "columnar".

In cases where the mesh shape is columnar (that is, cases where the mesh aspect ratio is not within the range of 1 to 3), the mesh aspect ratio is preferably 4 or 5 in view of bone bonding. In cases where the mesh aspect ratio is not more than 5, the level of bone bonding tends to be high.

The mesh shape is preferably spherical (having a mesh aspect ratio of 1 to 3) in view of tissue repair ability.

In cases where the mesh shape is columnar, the mesh pore diameter in the gelatin-containing intermediate is preferably 10 μm or larger, more preferably 50 μm or larger, still more preferably 100 μm or larger, in view of bone bonding. The upper limit of the mesh pore diameter in the gelatin-containing intermediate is not limited, and the pore size is preferably 2500 μm or smaller, more preferably 1000 μm or smaller, in view of stability of strength of the substance.

In cases where the longitudinal diameter is 20 μm or larger, cells are more likely to enter the inside of the mesh structure, and, in cases where the longitudinal diameter is 2500 μm or smaller, biocompatibility tends to be high.

In cases where the mesh shape is spherical, the mesh pore diameter in the gelatin-containing intermediate is preferably 10 μm or larger, more preferably 50 μm or more, still more preferably 100 μm or more, in view of bone bonding. The upper limit of the mesh pore diameter in the gelatin-containing intermediate is not limited, and the pore diameter is generally preferably 2500 μm or smaller, more preferably 1000 μm or smaller in view of bone bonding. When the mesh shape is spherical, in cases where the mesh pore diameter (longitudinal diameter, in cases where the aspect ratio is more than 1) is 10 μm or larger, cells are more likely to enter the inside of the mesh structure, and, in cases where the pore size is 2500 μm or smaller, biocompatibility tends to be high.

The porosity of the gelatin-containing intermediate is preferably from 80% to 99.99%, more preferably from 95.01% to 99.9%. The porosity is determined based on the bulk density (p) and the true density (pc), according to: porosity $(P = (1 - \rho / \rho c) \times 100$ (%)). The bulk density (p) is calculated from the dry mass and the volume, and the true density (pc) may be determined by the Gay-Lussac pycnometer method.

(b) Drying Step

In the drying step, the gelatin-containing intermediate is dried.

Examples of the drying method include fluidized bed drying, film drying, spray drying, and freeze drying. Freeze drying is preferably used.

In terms of the conditions for freezing, conditions normally used for freeze-drying of protein may be employed as they are. The period of freeze-drying may be, for example, from 0.5 hour to 300 hours. The freeze dryer that may be used is not limited.

(c) Cross-linking Step

In the cross-linking step, which is carried out before or after the drying step, cross-linking of the recombinant gelatin is carried out.

The method of cross-linking that is used is thermal cross-linking.

The cross-linking temperature applied to the thermal cross-linking method is preferably from 100°C to 200°C, more preferably from 110°C to 180°C, still more preferably from 120°C to 160°C. By employing the thermal cross-linking method, use of a cross-linking agent can be avoided. In cases where the cross-linking temperature is 100°C or higher, the cross-linking can be sufficiently allowed to proceed. On the other hand, in cases where the cross-linking temperature is 200°C or lower, side reactions other than cross-linking do not occur, which is preferred.

The atmosphere in which the thermal cross-linking treatment is carried out is not limited as long as the concentrations of oxygen and water vapor can be kept sufficiently low. The treatment is preferably carried out in an inert gas, or under vacuum at 5 kPa or less. The pressure of the inert gas is not limited, and the treatment is preferably carried out at a pressure of 0.15 MPa or lower. The type of the inert gas is also not limited, and nitrogen is preferably used.

The period of cross-linking can be appropriately selected by those skilled in the art depending on properties of the gelatin to be subjected to the cross-linking and the subject tissue, using as an index the acid degradability described below. As the period of cross-linking increases, and/or as the cross-linking temperature decreases, acid degradability decreases.

For example, in terms of acid degradability, the repair material for bone tissues preferably shows a residual ratio of 80% or lower by mass after 5 hours of degradation treatment using 1 mol/L (liter) hydrochloric acid. The residual ratio is more preferably from 10% to 70% after 3 hours of the treatment. In cases where the polypeptide of SEQ ID NO:1 is used as the gelatin to be subjected to cross-linking, the residual ratio can be achieved by from 4 to 20 hours of treatment at a temperature of from 120°C to 150°C in an inert gas of from vacuum (including 0 MPa) to 0.15 MPa.

The thermal cross-linking method is preferably carried out after the drying step in view of the shape and the tissue repair ability of the tissue repair material obtained.

[Acid Degradability]

Acid degradability of the tissue repair material of the invention is measured as follows. The acid degradability can be represented as an acid degradation rate. The mass of the microtube for measurement (hereinafter referred to as tube) is measured (A). After weighing 5.0 (±0.2) mg of a tissue repair material (B), the material is placed in the tube for measurement. To the tube containing the tissue repair material, 1.0 ml of 1 mol/L HCl is added, and the resulting mixture is shaken in a shaking incubator (HB-80 (Taitec Corporation), number of times of shaking per 1 minute: 60) at 37°C for a predetermined period. Thereafter, the tube is placed on ice to stop the reaction, and centrifuged at 10,000×g for 1 minute in a centrifuge whose temperature was preliminarily set to 4°C. After confirming precipitation of the tissue repair material, the supernatant is removed by suction, and 1 ml of ultrapure water preliminarily cooled on ice is added to the tube, followed by performing centrifugation again under the same conditions as described above. After removing the supernatant by suction, ultrapure water is added again, and centrifugation is carried out again under the same conditions as described above. After removing the supernatant by suction, freeze-drying is carried out. After the drying, the tube is removed from the freeze dryer, and the cap of the tube is immediately closed in order to prevent absorption of moisture in the air by the tissue repair material. The mass of the whole tube is measured (C), and the residual ratio is calculated according to Calculation Equation (3) below. The acid degradability is calculated according to Calculation Equation (4) below.

$$\text{Residual ratio} = (C\text{-}A) / B \times 100 \ (\%) \ \dots \ (3)$$

$$\text{Acid degradation rate} = 100 - \text{residual ratio} \ (\%) \ \dots \ (4)$$

In the tissue repair material of the invention, the residual ratio is preferably 80% or lower after 5 hours of the degradation treatment. In cases where the residual ratio after 5 hours of the treatment is higher than 80%, the tissue regeneration rate tends to be low. In the tissue repair material of the invention, in view of maintenance of the space of the defect and replacement by regenerated bone, the residual ratio is more preferably 70% by mass or lower, still more preferably 60% by mass or lower after 3 hours of the treatment. In the tissue repair material of the invention, in view of maintenance of the space of the site where the material is embedded, the lower limit of the residual ratio is preferably 10% by mass or higher, more preferably 30% by mass or higher after 3 hours of the treatment.

[Tissue Repair Material]

The tissue repair material obtained by the production method of the invention is a porous tissue repair material that contains the cross-linked recombinant gelatin and has a water absorption rate of 100% by mass or higher. Such a tissue repair material can have a favorable tissue repair ability.

The water absorption rate of a tissue repair material means a ratio determined by placing 15 mg of the tissue repair material in a nylon mesh bag having a size of 3 cm × 3 cm to allow swelling in ion-exchanged water for 2 hours at 25°C, drying the resultant in the air for 10 minutes, and then performing calculation according to Formula (5) below.

$$\text{Water absorption rate} = (w2 - w1 - w0) / w0 \ldots (5)$$

In this formula, w0 represents the mass of the material before water absorption; w1 represents the mass of the empty bag after absorption of water; and w2 represents the mass of the whole bag containing the material after absorption of water.

The water absorption rate is preferably 100% or higher, more preferably 200% or higher, still more preferably 400% or higher in view of the tissue repair ability. The upper limit of the water absorption rate is not limited, and the rate is generally 9900% or lower, preferably 5000% or lower. For example, in cases where the water absorption rate is 100% or more, the bone regeneration rate tends to be favorable.

The tissue repair material is a porous body having voids derived from a predetermined mesh structure in the gelatin-containing intermediate.

In the present description, "porous body" means a material having a plurality of pores in a body, or a material obtained by shredding such a material. The pores inside the material may be communicating with each other, and part or all of the pores may open to the surface of the material.

Since the predetermined mesh structure of the gelatin-containing intermediate is favorably maintained even after the drying step (in some cases, after the cross-linking step), structural properties of the tissue repair material are almost the same as properties in the gelatin-containing intermediate.

Similarly to the gelatin-containing intermediate, the porosity of the tissue repair material is preferably from 80% to 99.99%, more preferably from 95.01% to 99.9%. The porosity is determined based on the bulk density (p) and the true density (pc), according to: porosity ($P = (1 - p / \rho c) \times 100$ (%)). The bulk density (p) is calculated from the dry mass and the volume, and the true density (pc) maybe determined by the Hubbard pycnometer method.

Similarly to the gelatin-containing intermediate, communicating pores may be formed in the tissue repair material. Because of the presence of communicating pores, the outside of the tissue repair material communicates with the deep part through voids communicating with each other. Therefore, cells can disperse or spread into the inside of the porous body when the cells contact with the outside of the tissue repair material. The pore diameter of each communicating pore is preferably 10 $\mu$m or larger in view of exertion of the function.

The tissue repair material obtained may then be pulverized into powder. By this, a tissue repair material in the form of a powder, which is excellent in convenience, can be obtained. The pulverization method applied to the pulverization is not limited, and a method usually used for pulverizing a freeze-dried product may be applied as it is.

The tissue repair material of the present invention can be specified by the bone regeneration rate or bone bonding, or the combination thereof.

In terms of the bone regeneration rate, the bone regeneration rate described above may be applied as it is. A bone regeneration rate of 22% or higher is sufficient for recovery of a tissue, and the rate is preferably n 25% or higher.

A rate of bone bonding of 20% or higher is sufficient for recovery of a tissue, and the rate is preferably 25% or higher.

The tissues that can be recovered using the tissue repair material of the invention are preferably hard tissues such as tooth and bone. In particular, the tissue repair material may be used for bone regeneration as a restorative material for tissues, a therapeutic agent, or the like. The tissue repair material of the invention may be used alone as a bone regeneration therapeutic agent. The disease is not limited as long as the therapy requires bone regeneration or bone formation. The tissue repair material of the invention may also be used in combination with cells for transplantation and/or a bone inducing agent, to provide a bone regeneration therapeutic agent. Examples of the bone inducing agent include, but are not limited to, BMP (bone morphogenetic factor) and bFGF (basic fibroblast growth factor).

Since the invention can provide the tissue repair material having favorable tissue repair ability, a method of repairing a tissue and a method of treatment of a disease or the like accompanied by tissue damage are also included in the invention. The scope of the invention is defined by the claims. Any references in the description to methods of treatment refer to the tissue repair materials of the present invention for use in a method for treatment of the human (or animal) body by therapy (or for diagnosis).

Specifically, the method of repairing a tissue in the invention comprises application of the tissue repair material to an area in which a subject tissue is lost or damaged, and also comprises another step, if necessary.

Further, the method of treatment of a damaged tissue in the present invention comprises application of the tissue repair material, or, in cases where the subject tissue is bone, application of the bone regeneration therapeutic agent, to an area where the subject tissue is lost or damaged; and another/other step(s), if necessary. Examples of the another step include application of cells for transplantation and/or the bone inducing agent to the area of application of the tissue repair material, before, after, or at the same time as, the application of the tissue repair material.

For example, the therapeutic method or repair method may be preferably applied to periodontal defect, implant defect, and the like in the maxillofacial area; and to the GBR method, ridge augmentation method, sinus lift method, and socket reservation method as preliminary treatments for implanting.

EXAMPLES

The invention is described below in detail by way of Examples. However, the invention is not limited at all by the Examples. Unless otherwise specified, "%" is by mass.

[Example 1]

As a recombinant gelatin, a recombinant peptide CBE3 was used to produce the tissue repair material of Example 1.
The CBE3 employed was the one described below (described in WO2008/103041).

CBE3
Molecular weight: 51.6 kD
Structure: GAP[(GXY)$_{63}$]$_3$G
Number of amino acids: 571
Number of RGD sequences: 12
Imino acid content: 33%
Almost 100% of the amino acids are contained in the repeating structure of GXY.

The amino acid sequence of CBE3 contains none of a serine residue, threonine residue, asparagine residue, tyrosine residue or cysteine residue.

CBE3 has an ERGD sequence.
Isoelectric point: 9.34
Amino acid sequence (SEQ ID NO:1)

GAP(GAPGLQGAPGLQGMPGERGAAGLPGPKGERGDAGPKGADGAPGAPGLQGMP

GERGAAGLPGPKGERGDAGPKGADGAPGKDGVRGLAGPIGPPGERGAAGLPGPKGE

RGDAGPKGADGAPGKDGVRGLAGPIGPPGPAGAPGAPGLQGMPGERGAAGLPGPKG

ERGDAGPKGADGAPGKDGVRGLAGPP)$_3$G

An aqueous gelatin solution containing the recombinant gelatin at 12% by mass was poured into an aluminum tray, and left to stand in a freezer at -20°C overnight, to obtain a frozen gelatin block. The gelatin block was freeze-dried to obtain a dry intermediate 1. The dry intermediate 1 was pulverized using a pulverizer, and the fraction that passed through a 710-μm mesh sieve but did not pass through a 500-μm mesh sieve was collected. The collected fraction was treated under reduced pressure at 160°C for 15 hours, to provide Sample 1.
The shape of the mesh structure observed and the average diameter in the longitudinal axis (longitudinal diameter) in the dry intermediate 1; and the water absorption rate, bone regeneration, and bone bonding in Sample 1; were evaluated as follows.

(1) Mesh Shape and Average Longitudinal Diameter in Dry Intermediate 1

The dry intermediate 1 was cut in the horizontal and vertical directions. Subsequently, each section was brought into close contact with an ink pad to perform staining, and an area of 2.0 mm × 2.0 mm was observed under a light microscope. In the observed area, among circumscribed rectangles to an area surrounded by the stained material, the circumscribed rectangle having the largest distance between two opposite sides of the rectangle was selected. The length of long side of the circumscribed rectangle having the largest distance between two opposite sides was measured for 50 mesh pores in the observation area in each of the horizontal section and the vertical section, and the average was defined as the average longitudinal diameter of meshes of the dry intermediate 1.
Between the longitudinal diameter of each mesh (average) in the horizontal section and the longitudinal diameter of each mesh (average) in the vertical section, the smaller value was defined as d1, and the other was defined as d2 to calculate the ratio d2/d1 (mesh aspect ratio). In cases where the mesh aspect ratio was from 1 to 3, the shape was regarded as "spherical", and in cases where mesh aspect ratio was not within this range, the shape was regarded as "columnar", to evaluate the mesh shape. The results are shown in Table 1.

(2) Water Absorption Rate

At 25°C, about 15 mg of Sample 1 was placed in a nylon mesh bag having a size of 3 cm × 3 cm to allow swelling in ion-exchanged water for 2 hours, followed by drying the resultant in the air for 10 minutes. The mass was measured in each stage, and the water absorption rate was calculated according to Formula (5). The results are shown in Table 1.

$$\text{Water absorption rate} = (w2 - w1 - w0) / w0 \dots (5)$$

In this formula, w0 represents the mass of the material before water absorption; w1 represents the mass of the empty bag after absorption of water; and w2 represents the mass of the whole bag containing the material after absorption of water.

(3) Evaluation of Bone Regeneration

In the parietal bone of SD rats (male, from 10 to 12 weeks old, from 0.3 kg to 0.5 kg), a circular bone defect having a diameter of 5 mm was prepared, and the prepared bone defect was filled with about 3.6 mg of Sample 1, followed by suture of the skin. On Week 4 after the operation, the bone mass of the rat parietal bone was measured using a micro-CT, and the ratio of the bone volume in the defect to the volume of the defect was determined as the bone regeneration rate (%). The results are shown in Table 1.

(4) Evaluation of Bone Bonding

In the parietal bone of SD rats (male, from 10 to 12 weeks old, from 0.3 kg to 0.5 kg), a circular bone defect having a diameter of 5 mm was prepared, and the prepared bone defect was filled with about 3.6 mg of Sample 1, followed by suture of the skin. On Week 4 after the operation, the rats were sacrificed by bleeding to death under anesthesia with pentobarbital, and the head was removed. The parietal bone containing the embedded portion was subjected to HE staining, and histological observation was carried out. The number of sites where the embedded Sample 1 and the new bone are in contact with each other without interposition of a fibrous tissue was counted, and evaluation was carried out as follows depending on the average number of contacting sites per site (bone bonding rate) of the embedded Sample 1. The results are shown in Table 1.

A bone bonding ratio of 30% or more: excellent (A)
A bone bonding ratio of from 20% to less than 30%: good (B)
A bone bonding ratio of less than 20%: bad (C)

[Example 2]

An aqueous gelatin solution containing 12% by mass of the CBE3 as used in Example 1 was poured into a stainless-steel tray, and left to stand in a freezer at -50°C for 8 hours, to obtain a frozen gelatin block. The gelatin block was freeze-dried to obtain a dry intermediate 2. The dry intermediate 2 was pulverized using a pulverizer, and the fraction that passed through a 710-$\mu$m mesh sieve but did not pass through a 500-$\mu$m mesh sieve was collected. The collected fraction was treated under reduced pressure at 160°C for 19 hours, to obtain Sample 2.
The shape of the mesh structure observed and the average diameter in the longitudinal axis (longitudinal diameter) in the dry intermediate 2; and the water absorption rate, bone regeneration, and bone bonding in Sample 2; were evaluated in the same manner as in Example 1.

[Example 3]

An aqueous gelatin solution containing 12% by mass of the CBE3 as used in Example 1 was stirred in a pot cooled at 9°C, using a T. K. Homodisper Type 2.5 at 1400 rpm, and left to stand in a freezer at -50°C for 8 hours, to obtain a frozen gelatin block. The stirring under these conditions corresponds to a stirring Froude number Fr of 2.22 and a stirring Reynolds number of 3,700. The gelatin block was freeze-dried to obtain a dry intermediate 3. The dry intermediate 3 was pulverized using a pulverizer, and the fraction that passed through a 710-$\mu$m mesh sieve but did not pass through a 500-$\mu$m mesh sieve was collected. The collected fraction was treated under reduced pressure at 160°C for 19 hours, to obtain Sample 3.

The shape of the mesh structure observed and the average diameter in the longitudinal axis (longitudinal diameter) in the dry intermediate 3; and the water absorption rate, bone regeneration, and bone bonding in Sample 3; were evaluated in the same manner as in Example 1.

[Example 4]

An aqueous gelatin solution containing, at a final concentration of 7.5% by mass, the CBE3 used in Example 1 was prepared, and this aqueous gelatin solution was poured into a stainless-steel tray. Thereafter, the solution was left to stand in a freezer at -80°C for 8 hours, to obtain a frozen gelatin block. The gelatin block was freeze-dried to obtain a dry intermediate 4. The dry intermediate 4 was pulverized using a pulverizer, and the fraction that passed through a 710-$\mu$m mesh sieve but did not pass through a 500-$\mu$m mesh sieve was collected. The collected fraction was treated under reduced pressure at 160°C for 19 hours, to obtain Sample 4.

The shape of the mesh structure observed and the average diameter in the longitudinal axis (longitudinal diameter) in the dry intermediate 4; and the water absorption rate, bone regeneration, and bone bonding in Sample 4; were evaluated in the same manner as in Example 1.

[Example 5]

An aqueous gelatin solution containing, at a final concentration of 7.5% by mass, the CBE3 used in Example 1 was prepared, and this aqueous gelatin solution was poured into an aluminum container. Thereafter, the container was placed, via a heat-insulating material, on a duralumin block pre-cooled in a freezer at -40°C. The container was then left to stand for 1 hour, to obtain a frozen gelatin block. The gelatin block was freeze-dried to obtain a dry intermediate 5. The dry intermediate 5 was pulverized using a pulverizer, and the fraction that passed through a 710-$\mu$m mesh sieve but did not pass through a 500-$\mu$m mesh sieve was collected. The collected fraction was treated under a nitrogen atmosphere at 0.08 MPa at 137°C for 7 hours, to obtain Sample 5.

The shape of the mesh structure observed and the average diameter in the longitudinal axis (longitudinal diameter) in the dry intermediate 5; and the water absorption rate, bone regeneration, and bone bonding in Sample 5; were evaluated in the same manner as in Example 1.

[Comparative Example 1]

An aqueous gelatin solution containing 12% by mass of the CBE3 as used in Example 1 was poured into a polypropylene tray, and left to stand in a refrigerator (4°C) for 1 week, to obtain a dry gelatin block. The gelatin block was freeze-dried to obtain a dry intermediate 6. The dry intermediate 6 was pulverized using a pulverizer, and the fraction that passed through a 710-$\mu$m mesh sieve but did not pass through a 500-$\mu$m mesh sieve was collected. The collected fraction was treated under reduced pressure at 160°C for 19 hours, to obtain Sample 6.

The shape of the mesh structure observed and the average diameter in the longitudinal axis (longitudinal diameter) in the dry intermediate 6; and the water absorption rate, bone regeneration, and bone bonding in Sample 6; were evaluated in the same manner as in Example 1.

[Table 1]

|  | Shape | Longitudinal diameter ($\mu$m) | Water absorption rate (%) | Acid degradation rate (%) (5 hours of treatment) | Bone regeneration rate (%) | Evaluation of bone bonding |
|---|---|---|---|---|---|---|
| Example 1 | Columnar | 2324 | 119 | 33 | 25 | B |
| Example 2 | Spherical | 583 | 583 | 29 | 33 | A |
| Example 3 | Spherical | 325 | 1351 | 24 | 40 | A |
| Example 4 | Spherical | 65 | 790 | 34 | 34 | A |
| Example 5 | Spherical | 42 | 1363 | 97 | 54 | A |

(continued)

|  | Shape | Longitudinal diameter (μm) | Water absorption rate (%) | Acid degradation rate (%) (5 hours of treatment) | Bone regeneration rate (%) | Evaluation of bone bonding |
|---|---|---|---|---|---|---|
| Comparative Example 1 | None | - | 73 | 22 | 21 | C |

Thus, since the tissue repair materials according to the Examples of the invention were obtained by freeze-drying a gelatin intermediate having a predetermined mesh structure, the materials had high water absorption rates and high bone regeneration rates, and were tissue repair materials that can induce favorable bone bonding.

Accordingly, the invention can provide a method of producing a tissue repair material, which method enables production of a tissue repair material having favorable tissue regeneration ability.

The above description on exemplary embodiments of the invention was done for the purposes of exemplification and explanation, and intends neither to provide comprehensive description nor to limit the invention to the modes disclosed. As is evident, many modifications or changes are obvious to those skilled in the art. The embodiments were selected and described such that the embodiments best explain the principle and practical application of the invention and allow those skilled in the art other than the present inventors to understand the invention together with various embodiments and various modifications suitable for specific uses that can be assumed. The scope of the invention is intended to be specified by the claims below.

**Claims**

1. A method of producing a tissue repair material comprising:

    (a) obtaining a gelatin-containing intermediate that contains a gelatin and has a mesh structure, using a gelatin solution in which the gelatin is dissolved in an aqueous medium by stirring the gelatin solution to generate air bubbles in the gelatin solution, and then allowing the gelatin solution to gel by cooling at a temperature of from 1 °C to 20 °C without a chemical cross-linking agent;
    (b) drying the gelatin-containing intermediate; and
    (c) cross-linking the gelatin before or after the drying of the gelatin-containing intermediate by thermal cross-linking.

2. The method of producing a tissue repair material according to claim 1, wherein the drying is freeze-drying.

3. The method of producing a tissue repair material according to claim 1 or 2, wherein the gelatin is a recombinant gelatin.

4. The method of producing a tissue repair material according to any one of claims 1 to 3, wherein the gelatin-containing intermediate has a mesh pore diameter of 10 $\mu$m or larger.

5. The method of producing a tissue repair material according to any one of claims 1 to 4, comprising obtaining the gelatin-containing intermediate by freezing the gelatin solution at a temperature of from -100°C to -10°C.

6. The method of producing a tissue repair material according to any one of claims 1 to 5, wherein the cross-linking of the gelatin is carried out by heating under conditions at a temperature of from 100°C to 200°C after the drying.

7. The method of producing a tissue repair material according to any one of claims 1 to 6, wherein the cross-linking of the gelatin is carried out by heating, and the cross-linking of the gelatin is carried out such that the acid degradation rate is 20% or higher when 1 ml of 1 mol/L hydrochloric acid is added to 5 mg of the produced tissue repair material followed by treatment at 37°C for 5 hours.

8. The method of producing a tissue repair material according to any one of claims 3 to 7, wherein the recombinant gelatin comprises a repeating unit of a sequence represented by Gly-X-Y (wherein each of X and Y represents an arbitrary amino acid residue), and a cell adhesion signal.

9. The method of producing a tissue repair material according to claim 8, wherein the recombinant gelatin has a sequence represented by A-[(Gly-X-Y)$_n$]$_m$-B (wherein each of X and Y represents an arbitrary amino acid residue; m represents an integer from 2 to 9; n represents an integer from 3 to 10; and A and B each independently represent an arbitrary amino acid residue or amino acid sequence) and a cell adhesion signal.

10. The method of producing a tissue repair material according to claim 8 or 9, wherein the recombinant gelatin has a sequence represented by Gly-Ala-Pro-[(Gly-X-Y)$_{63}$]$_3$-Gly (wherein each of the 63 Xs independently represents an arbitrary amino acid residue; each of the 63 Ys independently represents an arbitrary amino acid residue; and each of the 3 units of (Gly-X-Y)$_{63}$ may be the same as or different from each other) and a cell adhesion signal.

11. The method of producing a tissue repair material according to any one of claims 3 to 10, wherein the recombinant gelatin is any of (A) to (C) below:

(A) a polypeptide represented by SEQ ID NO:1;
(B) a polypeptide having a partial sequence with a sequence identity of 80% or higher to the partial amino acid sequence consisting of from the 4th to 192nd amino acid residues in the amino acid sequence of (A), and having tissue repair ability; or
(C) a polypeptide consisting of the same amino acid sequence as the amino acid sequence of (A) except that one or several amino acids are deleted, substituted and/or added, and having tissue repair ability.

12. The method of producing a tissue repair material according to any one of claims 1 to 11, wherein the tissue is bone.

13. A tissue repair material obtained by the production method according to any one of claims 1 to 12, which comprises the cross-linked gelatin and is a porous material having a water absorption rate of 100% or higher by mass.

**Patentansprüche**

1. Verfahren zur Herstellung eines Gewebereparaturmaterials, umfassend:

(a) Erhalten eines gelatinehaltigen Zwischenprodukts, das eine Gelatine enthält und eine Maschenstruktur (mesh structure) aufweist, unter Verwendung einer Gelatinelösung, in welcher die Gelatine in einem wässrigen Medium durch Rühren der Gelatinelösung gelöst wird, um Luftblasen in der Gelatinelösung zu erzeugen, und dann Gelierenlassen der Gelatinelösung durch Abkühlen bei einer Temperatur von 1°C bis 20°C ohne ein chemisches Vernetzungsmittel;
(b) Trocknen des gelatinehaltigen Zwischenprodukts; und
(c) Vernetzung der Gelatine vor oder nach dem Trocknen des gelatinehaltigen Zwischenprodukts durch thermische Vernetzung.

2. Verfahren zur Herstellung eines Gewebereparaturmaterials gemäß Anspruch 1, wobei das Trocknen Gefriertrocknen ist.

3. Verfahren zur Herstellung eines Gewebereparaturmaterials gemäß Anspruch 1 oder 2, wobei die Gelatine eine rekombinante Gelatine ist.

4. Verfahren zur Herstellung eines Gewebereparaturmaterials gemäß irgendeinem der Ansprüche 1 bis 3, wobei das gelatinehaltige Zwischenprodukt einen Maschenporendurchmesser von 10 $\mu$m oder größer aufweist.

5. Verfahren zur Herstellung eines Gewebereparaturmaterials gemäß irgendeinem der Ansprüche 1 bis 4, umfassend Erhalten des gelatinehaltigen Zwischenprodukts durch Einfrieren der Gelatinelösung bei einer Temperatur von -100°C bis -10°C.

6. Verfahren zur Herstellung eines Gewebereparaturmaterials gemäß irgendeinem der Ansprüche 1 bis 5, wobei die Vernetzung der Gelatine durch Erhitzen unter Bedingungen bei einer Temperatur von 100°C bis 200°C nach dem Trocknen durchgeführt wird.

7. Verfahren zur Herstellung eines Gewebereparaturmaterials gemäß irgendeinem der Ansprüche 1 bis 6, wobei die Vernetzung der Gelatine durch Erhitzen durchgeführt wird und die Vernetzung der Gelatine so durchgeführt wird, dass die Säureabbaurate 20% oder höher ist, wenn 1 ml 1 mol/L Salzsäure zu 5 mg des hergestellten Gewebereparaturmaterials hinzugefügt wird, gefolgt von einer Behandlung bei 37°C für 5 Stunden.

8. Verfahren zur Herstellung eines Gewebereparaturmaterials gemäß irgendeinem der Ansprüche 3 bis 7, wobei die rekombinante Gelatine eine Wiederholungseinheit einer durch Gly-X-Y repräsentierten Sequenz (wobei X und Y jeweils einen beliebigen Aminosäurerest repräsentieren) und ein Zelladhäsionssignal umfasst.

9. Verfahren zur Herstellung eines Gewebereparaturmaterials gemäß Anspruch 8, wobei die rekombinante Gelatine eine Sequenz aufweist, repräsentiert durch A-[(Gly-X-Y)$_n$]$_m$-B (wobei jedes von X und Y einen beliebigen Aminosäurerest repräsentieren; m eine ganze Zahl von 2 bis 9 repräsentiert; n eine ganze Zahl von 3 bis 10 repräsentiert; und A und B jeweils unabhängig voneinander einen beliebigen Aminosäurerest oder eine beliebige Aminosäuresequenz repräsentieren) und ein Zelladhäsionssignal aufweist.

10. Verfahren zur Herstellung eines Gewebereparaturmaterials gemäß Anspruch 8 oder 9, wobei die rekombinante Gelatine eine Sequenz aufweist, repräsentiert durch Gly-Ala-Pro-[(Gly-X-Y)$_{63}$]$_3$-Gly (wobei jedes der 63 X unabhängig voneinander einen beliebigen Aminosäurerest repräsentiert; jedes der 63 Y unabhängig voneinander einen beliebigen Aminosäurerest repräsentiert; und jede der 3 Einheiten von (Gly-X-Y)$_{63}$ gleich oder verschieden voneinander sein kann) und ein Zelladhäsionssignal aufweist.

11. Verfahren zur Herstellung eines Gewebereparaturmaterials gemäß irgendeinem der Ansprüche 3 bis 10, wobei die rekombinante Gelatine eine der folgenden (A) bis (C) ist:

(A) ein Polypeptid, repräsentiert durch SEQ ID NO:1;
(B) ein Polypeptid mit einer Teilsequenz mit einer Sequenzidentität von 80 % oder höher zu der Teilaminosäuresequenz, bestehend aus dem 4. bis 192. Aminosäurerest in der Aminosäuresequenz von (A), und mit Gewebereparaturfähigkeit; oder
(C) ein Polypeptid, das aus der gleichen Aminosäuresequenz wie die Aminosäuresequenz von (A) besteht, mit der Ausnahme, dass eine oder mehrere Aminosäuren deletiert, substituiert und/oder hinzugefügt werden, und das die Fähigkeit zur Gewebereparatur besitzt.

12. Verfahren zur Herstellung eines Gewebereparaturmaterials gemäß irgendeinem der Ansprüche 1 bis 11, wobei das Gewebe Knochen ist.

13. Gewebereparaturmaterial, erhalten durch das Herstellungsverfahren gemäß irgendeinem der Ansprüche 1 bis 12, welches die vernetzte Gelatine umfasst und ein poröses Material mit einer Wasserabsorptionsrate von 100 Massen-% oder mehr ist.

**Revendications**

1. Procédé de production d'un matériau de réparation tissulaire comprenant les étapes consistant à :

(a) obtenir un intermédiaire contenant de la gélatine qui contient une gélatine et a une structure maillée, en utilisant une solution de gélatine dans laquelle la gélatine est dissoute dans un milieu aqueux en agitant la solution de gélatine pour générer des bulles d'air dans la solution de gélatine, puis en laissant la solution de gélatine se gélifier par refroidissement à une température de 1 °C à 20 °C sans agent de réticulation chimique ;

(b) sécher l'intermédiaire contenant de la gélatine ; et

(c) réticuler la gélatine avant ou après le séchage de l'intermédiaire contenant de la gélatine par réticulation thermique.

2. Procédé de production d'un matériau de réparation tissulaire selon la revendication 1, dans lequel le séchage est une lyophilisation.

3. Procédé de production d'un matériau de réparation tissulaire selon la revendication 1 ou 2, dans lequel la gélatine est une gélatine recombinante.

4. Procédé de production d'un matériau de réparation tissulaire selon l'une quelconque des revendications 1 à 3, dans lequel l'intermédiaire contenant de la gélatine a un diamètre de pore de maille de 10 $\mu$m ou plus.

5. Procédé de production d'un matériau de réparation tissulaire selon l'une quelconque des revendications 1 à 4, comprenant l'étape consistant à obtenir l'intermédiaire contenant de la gélatine par congélation de la solution de gélatine à une température de -100 °C à -10 °C.

6. Procédé de production d'un matériau de réparation tissulaire selon l'une quelconque des revendications 1 à 5, dans lequel la réticulation de la gélatine est effectuée par chauffage dans des conditions à une température de 100 °C à 200 °C après le séchage.

7. Procédé de production d'un matériau de réparation tissulaire selon l'une quelconque des revendications 1 à 6, dans lequel la réticulation de la gélatine est effectuée par chauffage, et la réticulation de la gélatine est effectuée de telle sorte que le taux de dégradation d'acide soit de 20 % ou plus lorsque 1 ml d'acide chlorhydrique à 1 mol/L est ajouté à 5 mg du matériau de réparation tissulaire produit, suivie d'un traitement à 37°C pendant 5 heures.

8. Procédé de production d'un matériau de réparation tissulaire selon l'une quelconque des revendications 3 à 7, dans lequel la gélatine recombinante comprend une unité répétitive d'une séquence représentée par Gly-X-Y (dans lequel chacun de X et Y représente un résidu d'acide aminé arbitraire), et un signal d'adhésion cellulaire.

9. Procédé de production d'un matériau de réparation tissulaire selon la revendication 8, dans lequel la gélatine recombinante a une séquence représentée par A-$[(Gly-X-Y)_n]_m$-B (dans lequel chacun de X et Y représente un résidu d'acide aminé arbitraire ; m représente un entier de 2 à 9 ; n représente un entier de 3 à 10 ; et A et B représentent chacun indépendamment un résidu d'acide aminé arbitraire ou une séquence d'acides aminés) et un signal d'adhésion cellulaire.

10. Procédé de production d'un matériau de réparation tissulaire selon la revendication 8 ou 9, dans lequel la gélatine recombinante a une séquence représentée par Gly-Ala-Pro-$[(Gly-X-Y)_{63}]_3$-Gly (dans lequel chacun des 63 X représente indépendamment un résidu d'acide aminé arbitraire; chacun des 63 Y représente indépendamment un résidu d'acide aminé arbitraire ; et chacune des 3 unités de $(Gly-X-Y)_{63}$ peut être mutuellement identique ou différente) et un signal d'adhésion cellulaire.

11. Procédé de production d'un matériau de réparation tissulaire selon l'une quelconque des revendications 3 à 10, dans laquelle la gélatine recombinante est l'un quelconque des (A) à (C) ci-dessous :

(A) un polypeptide représenté par SEQ ID NO : 1 ;

(B) un polypeptide ayant une séquence partielle avec une identité de séquence de 80 % ou plus par rapport à la séquence d'acides aminés partielle consistant en du 4e au 192e résidus d'acides aminés dans la séquence d'acides aminés de (A), et ayant une capacité de réparation tissulaire ; ou

(C) un polypeptide consistant en la même séquence d'acides aminés que la séquence d'acides aminés de (A) si ce n'est qu'un ou plusieurs acides aminés sont supprimés, substitués et/ou ajoutés, et ayant une capacité de réparation tissulaire.

12. Procédé de production d'un matériau de réparation tissulaire selon l'une quelconque des revendications 1 à 11, dans lequel le tissu est de l'os.

13. Matériau de réparation tissulaire obtenu par le procédé de production selon l'une quelconque des revendications 1 à 12, qui comprend la gélatine réticulée et est un matériau poreux ayant un taux d'absorption d'eau de 100 % ou plus en masse.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011027850 A
- JP H8196618 A
- EP 1014176 A2
- US 6992172 B
- WO 200485473 A
- WO 2008103041 A
- JP 010519293 A
- JP 2010519252 A
- JP 2010518833 A
- JP 2010519251 A
- WO 2010128672 A
- WO 2010147109 A
- JP 2012055020 A